# EUROPÄISCHE PATENTANMELDUNG

(11) **EP 2 174 641 A1**
(43) Veröffentlichungstag der Anmeldung: **14.04.2010**
(21) Anmeldenummer: 08017705.8
(22) Anmeldetag: 09.10.2008
(51) Int. Cl.: A61K 8/31, A61K 8/49, A61Q 19/00

(54) **Ölkörpermischungen, enthaltend Derivate des Isosorbids**

(71) Anmelder: Cognis IP Management GmbH, 40589 Düsseldorf (DE)
(72) Erfinder: Ansmann, Achim., 40699 Erkrath (DE); Prinz, Daniela., 41542 Dormagen (DE); Dierker, Markus., 40593 Düsseldorf (DE)

(57) **Zusammenfassung**

Die Anmeldung betrifft Ölkörper Mischung, enthaltend (A) mindestens einen flüchtigen Kohlenwasserstoff und (B) mindestens einen Diacylester des Isosorbids der folgenden Formel (I) 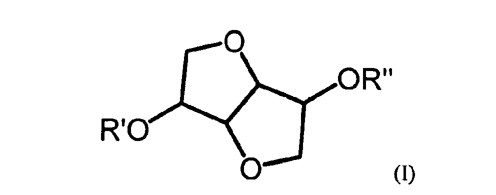 worin R' und R" unabhängig voneinander einen linearen oder verzweigten, gesättigten oder ungesättigten Acylrest mit 1 bis 24 C Atomen darstellt.

## Beschreibung

Die Erfindung betrifft Ölkörpermischungen sowie kosmetische Zubereitungen, enthaltend Ölkörper.

Im Bereich kosmetischer Zubereitungen für die Haut- und Haarpflege werden vom Verbraucher eine Vielzahl von Anforderungen gestellt: Abgesehen von den reinigenden und pflegenden Effekten, die den Anwendungszweck bestimmen, wird Wert auf so unterschiedliche Parameter wie höchstmögliche dermatologische Verträglichkeit, gute rückfettende Eigenschaften, elegantes Erscheinungsbild, optimaler sensorischer Eindruck und Lagerstabilität gelegt.

Zubereitungen, die zur Reinigung und Pflege der menschlichen Haut und der Haare eingesetzt werden, enthalten in der Regel neben einer Reihe von oberflächenaktiven Substanzen, vor allem Ölkörper und Wasser. Als Ölkörper/Emollients werden beispielsweise Kohlenwasserstoffe, Esteröle sowie pflanzliche und tierische Öle/Fette/Wachse eingesetzt. Um die hohen Anforderungen des Marktes bezüglich sensorischer Eigenschaften und optimaler dermatologischer Verträglichkeit zu erfüllen, werden kontinuierlich neue Ölkörper und Emulgator-Gemische entwickelt und getestet. Zur Herstellung kosmetischer Zubereitungen werden eine Vielzahl von natürlichen und synthetischen Ölen, beispielsweise Mandel- oder Avocadoöl, Esteröle, Ether, Alkylcarbonate, Kohlenwasserstoffe sowie Silikonöle eingesetzt. Eine wesentliche Aufgabe der Ölkomponenten ist es, neben der pflegenden Wirkung, die in unmittelbarem Zusammenhang mit der Hautfettung steht, dem Konsumenten ein nichtklebriges, möglich rasch eintretendes und lang anhaltendes Gefühl der Hautglätte und -geschmeidigkeit zu vermitteln.

Ölkörper sind wesentlicher Bestandteil der Mehrzahl der kosmetischen Mittel. Obgleich eine Vielzahl von Ölkörpern im Stand der Technik bekannt sind, besteht weiterhin Bedarf nach neuen Ölkörpern, die hinsichtlich Ihrer Verträglichkeit und Formulierbarkeit den bekannten Ölkörpern vergleichbar sind und darüber hinaus vorteilhafte sensorische Eigenschaften aufweisen. Bekannte Silikonöle sind hinsichtlich Ihrer Formulierbarkeit (v.a. hinsichtlich der Emulgierbarkeit) nur eingeschränkt einsetzbar. Es besteht insbesondere ein Bedarf nach Ölkörpem, welche eine den Silikonölen vergleichbare Sensorik auf der Haut erzeugen. Es hat nicht an Versuchen gefehlt, diese "Silikonsensorik" durch silikonfreie Formulierungen zu erreichen, wie z.B. in DE 10361568 beschrieben.

Die Aufgabe der vorliegenden Erfindung hat somit darin bestanden, alternative Ölkörper bereit zu stellen, die in kosmetischen Zubereitungen eingesetzt werden können. Von besonderem Interesse waren Ölkörper, welche in kosmetischen Zubereitungen eingesetzt werden können und diesen - ohne den Zusatz von Silikonölen eine "silikonartige Sensorik" verleihen. Als sensorische Parameter sind hierbei zu nennen: Leichtigkeit, "nicht-fettendes Hautgefühl", Weichheit, Spreitvermögen, Absorption, Verteilbarkeit, Öligkeit. Gleichzeitig war wünschenswert, dass diese Ölkörper eine gegenüber Silikonölen verbesserte Emulgierbarkeit aufweisen.

Gegenstand der Erfindung sind Ölkörpermischung, enthaltend (A) mindestens einen flüchtigen Kohlenwasserstoff und (B) mindestens einen Diacylester des Isosorbids der folgenden Formel (I) 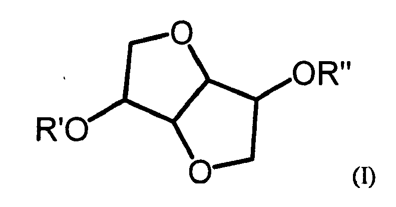 worin R' und R" unabhängig voneinander einen linearen oder verzweigten, gesättigten oder ungesättigten Acylrest mit jeweils insgesamt 1 bis 24 C Atomen darstellen.

Die Ölkörpermischungen eignen sich insbesondere als Silikonersatz in kosmetischen Zubereitungen.

Als "Ölkörper" im Sinne der Erfindung werden alle wasserunlöslichen Substanzen bezeichnet. Als "wasserunlöslich" werden Substanzen definiert, welche bei Raumtemperatur (23 °C) mit Wasser nicht mischbar sind.

Die erfindungsgemäßen Ölkörpermischungen können neben den Komponenten (A) und (B) weitere lipophile Komponenten enthalten. Diese weiteren lipophilen Komponenten können beispielsweise Öle, Fette, Wachse sowie beliebige Mischungen daraus sein. Diese weiteren lipophilen Komponenten können beispielsweise lipophile Emulgatoren oder liphophile Wirkstoffe, wie beispielsweise Vitamine, sein.

Unter dem Begriff Öle werden wasserunlösliche, bei 30 °C flüssige, organische Verbindungen mit relativ niedrigem Dampfdruck bezeichnet. Das gemeinsame Merkmal der Öle ist nicht ihre übereinstimmende chemische Konstitution, sondere ihre ähnliche physikalische Konsistenz. Unter Fetten versteht man Triacylglycerine, also die Dreifachester von Fettsäuren mit Glycerin. Bevorzugt enthalten sie gesättigte, unverzweigte und unsubstituierte Fettsäurereste. Hierbei kann es sich auch um Mischester, also um Dreifachester aus Glycerin mit verschiedenen Fettsäuren handeln. Unter dem Begriff Wachs werden üblicherweise alle natürlichen oder künstlich gewonnenen Stoffe und Stoffgemische mit folgenden Eigenschaften verstanden: sie sind von fester bis brüchig harter Konsistenz, grob bis feinteilig, durchscheinend bis trüb und schmelzen oberhalb von 30°C ohne Zersetzung. Sie sind schon wenig oberhalb des Schmelzpunktes niedrigviskos und nicht fadenziehend und zeigen eine stark temperaturabhängige Konsistenz und Löslichkeit.

In einer bevorzugten Ausführungsform der Erfindung enthalten die Ölkörpermischungen 60 Gew.-% oder mehr, 70 Gew.-% oder mehr, insbesondere 80 Gew.-% oder mehr, besonders bevorzugt 90 Gew.-% oder mehr, insbesondere 95 Gew.-% oder mehr der Komponenten (A) und (B).

In einer Ausführungsform der Erfindung, enthält die Ölkörpermischung weniger als 5 Gew.-%, insbesondere weniger als 3 Gew.-%, insbesondere weniger als 1 Gew.-% Silikonöle. In einer Ausführungsform der Erfindung enthält die Ölkörper Mischung keine Silikonöle.

In einer Ausführungsform der Erfindung ist die Menge an (A) größer gleich 50 Gew.-% bezogen auf die Gesamtmenge von (A) und (B), insbesondere größer gleich 60 Gew.-%, bevorzugt größer gleich 70 Gew.-%. Diese Ölkörper Mischungen zeichnen sich durch Ihre Sensorik aus, insbesondere dadurch, dass sie eine leichte Verteilbarkeit auf der Haut ermöglichen und besonders schnell in die Haut einziehen.

In einer bevorzugten Ausführungsform der Erfindung enthalten die Ölkörpermischungen 60 Gew.-% oder mehr, 70 Gew.-% oder mehr, insbesondere 80 Gew.-% oder mehr, besonders bevorzugt 90 Gew.-% oder mehr, insbesondere 95 Gew.-% oder mehr der Komponenten (A) und (B) und die Menge an (A) ist größer gleich 50 Gew.-% bezogen auf die Gesamtmenge von (A) und (B), insbesondere größer gleich 60 Gew.-%, bevorzugt größer gleich 70 Gew.-%

Die Erfindung umfasst Ölkörpermischungen welche mindestens einen flüchtigen Kohlenwasserstoff (A) enthalten und mindestens einen Diester des Isosorbids.

Die erfindungsgemäßen Ölkörpermischungen lassen sich durch einfaches Mischen der jeweiligen Komponenten (A) und (B) sowie gegebenenfalls weiterer lipophiler Komponenten, gegebenenfalls bei erhöhten Temperaturen herstellen.

Gegenstand der Erfindung sind weiterhin kosmetische Zubereitungen, enthaltend
(A) mindestens einen flüchtigen Kohlenwasserstoff und
(B) mindestens einen Diacylester des Isosorbids

In einer Ausführungsform der Erfindung enthalten die kosmetischen Zubereitungen weniger als 5 Gew.-%, insbesondere weniger als 3 Gew.-%, insbesondere weniger als 1 Gew.-% Silikonöle. In einer Ausführungsform der Erfindung enthalten die kosmetischen Zubereitungen keine Silikonöle. Vielmehr ist einer der Gegenstände der der vorliegenden technischen Lehre darin zu sehen, die Ölkörpermischungen und insbesondere die beanspruchten Isosorbidderivate als Ersatz für Silikonöle in kosmetischen Zubereitungen zu verwenden.

Besonders bevorzugt enthalten die kosmetischen Zubereitungen die Komponenten (A) und (B) in solchen Mengen, dass die Menge an (A) größer gleich 50 Gew.-% bezogen auf die Gesamtmenge von (A) und (B), insbesondere größer gleich 60 Gew.-%, bevorzugt größer gleich 70 Gew.-% ist.

In einer bevorzugten Ausführungsform der Erfindung enthält die kosmetische Zubereitung neben (A) und (B) weniger als 40 Gew.-%, insbesondere weniger als 30 Gew.-%, insbesondere weniger als 20 Gew.-%, bevorzugt weniger als 10 Gew.-% weiterer lipophiler Komponenten.

Die erfindungsgemäßen kosmetischen Zubereitungen können ausschließlich aus lipophilen Komponenten bestehen, z.B. in Form von Hautöl, Babyöl, Reinigungsöl. Die erfindungsgemäßen kosmetischen Zubereitungen können aber auch Wasser, Emulgatoren, Tenside sowie weitere übliche kosmetischen Inhaltsstoffe enthalten, wie beispielsweise Konservierungsmittel, biogene Wirkstoffe, UV-Lichtschutzfaktoren, Verdickungsmittel, Überfettungsmittel, Stabilisatoren, Polymere, Antioxidantien, Deodorantien, Filmbildner, Quellmittel, Insektenrepellentien, Hydrotrope, Solubilisatoren, Parfümöle, Farbstoffe etc. Die Mengen der jeweiligen Zusätze richten sich nach der beabsichtigten Verwendung. Die kosmetischen Zubereitungen können neben den Komponenten (A) und (B) noch weitere lipophile Komponenten enthalten. Die Summe der Komponenten (A) und (B) sowie der weiteren lipophilen Komponenten liegt üblicherweise bei 1 bis 90, insbesondere 5 bis 50 Gew.-%. bezogen auf das Gesamtgewicht der kosmetischen Zubereitung.

Dementsprechend können die erfindungsgemäßen kosmetischen Zubereitungen beispielsweise in folgenden Formen vorliegen: sprühbare Zubereitungen für die Körperpflege (Deosprays, Sonnenschutzsprays usw.), Pflegeprodukte in Gel- oder Cremeform (W/O oder O/W Emulsionen), Wirkstoff-haltige Sprays, Gele oder Cremes, getränkte Pflegetücher oder Pads (Make-up-Entferner, Reinigungstücher usw.) und Ähnliches.

Als Komponente (A) wird mindestens ein flüchtiger Kohlenwasserstoff eingesetzt. Flüchtige Kohlenwasserstoffe sind solche Verbindungen, deren Siedepunkt bei Normaldruck (1013 mbar) kleiner 300 °C ist. Werden Mischungen aus verschiedenen Kohlenwasserstoffen eingesetzt, so ist maßgebend, dass der Siedepunkt der Hauptfraktion (d.h. 90% des Materials) kleiner 300 °C ist. In einer bevorzugten Ausführungsform werden als Komponenten (A) unverzweigte Kohlenwasserstoffe eingesetzt. In einer bevorzugten Ausführungsform der Erfindung werden als flüchtige Kohlenwasserstoffe (A) Kohlenwasserstoffe mit einer C Zahl von größer 6, insbesondere größer 8, bevorzugst größer 10 eingesetzt.
In einer bevorzugten Ausführungsform der Erfindung werden als flüchtige Kohlenwasserstoffe (A) gesättigte und ungesättigte, lineare und verzweigte, cyclische Kohlenwasserstoffe mit einer C Zahl von 6 bis 20, insbesondere mit einer C Zahl von 10 bis 16, eingesetzt. In einer besonders bevorzugten Ausführungsform der Erfindung werden als flüchtige Kohlenwasserstoffe (A) gesättigte, lineare Kohlenwasserstoffe mit einer C Zahl von 6 bis 20, insbesondere mit einer C Zahl von 10 bis 16, eingesetzt.

Geeignete flüchtige Kohlenwasserstoffe sind beispielsweise n-Decan, n-Undecan, n-Dodecan, n-Tridecan, n-Tetradecan, n-Pentadecan und n-Hexadecan, Isodecan, Isoundecan, Isododecan, Isotridecan, Isotetradecan, Isopentadecan, Isohexadecan, Diethyldecan, Diethyldodecan sowie Mineralöle (sofern sie das Kriterium der Flüchtigkeit erfüllen). In einer bevorzugten Ausführungsform wird als Komponente (A) mindestens eine Verbindung ausgewählt aus der Gruppe bestehend aus n-Decan, n-Undecan, n-Dodecan, n-Tridecan, n-Tetradecan, n-Pentadecan und n-Hexadecan eingesetzt.

Der Begriff "Mineralöle" ist eine Sammelbezeichnung für die aus mineralischen Rohstoffen (Erdöl, Braun- u. Steinkohlen, Holz, Torf) gewonnenen flüssigen Destillationsprodukte, die im Wesentlichen aus Gemischen von gesättigten Kohlenwasserstoffen bestehen. Solche Verbindungen sind als kosmetische Rohstoffe beispielsweise unter den Handelsnamen Halpasole (Fa. Haltermann) erhältlich. In einer bevorzugten Ausführungsform der Erfindung sind die Mischungen bzw. die kosmetischen Mittel, die die Mischungen enthalten frei von Mineralölen.

Geeignete flüchtige Kohlenwasserstoffe sind beispielsweise die in WO 03/35707 auf S.6, Z. 25 bis S.7, Z. 12 beschriebenen Poly-alpha-Olefine, auf die hier ausdrücklich Bezug genommen wird. Geeignete flüchtige Kohlenwasserstoffe sind beispielsweise die in der internationalen Anmeldung PCT/EP2006/001641 auf S.2 bis 6 beschriebenen Verbindungen, sofern sie das Kriterium der Flüchtigkeit erfüllen.

Die flüchtigen Kohlenwasserstoffe (A) können sowohl einzeln als auch in Mischungen untereinander eingesetzt werden. Da die Verwendung der Mischungen in kosmetischen Mitteln vorgesehen ist, sind solche Kohlenwasserstoffe bevorzugt, die nur einen geringen, oder vorzugsweise keinen vom Menschen wahrnehmbaren Eigengeruch aufweisen.

Die Isosorbidderivate (B) sind an sich bekannte Produkte, die durch Veresterung von Isosorbid mit Carbonsäuren und vorzugsweise Fettsäuren erhalten werden. Die Herstellung derartiger Verbindungen wird beispielsweise in der WO 2006/103338 A1 beschrieben. Die DE 19723732 A1 beschreibt Verfahren zur Herstellung von Monoacylderivaten des Isosorbids.

Die Isosorbidderivate im Sinne der vorliegenden technischen Lehre werden durch die Formel (I) beschrieben. Besonders bevorzugt sind dabei die Diacylderivate, die der Formel (II) folgen: 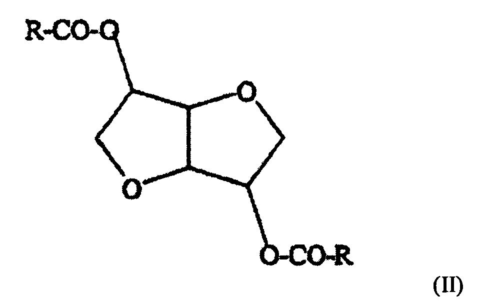 Die Reste R stehen dabei für gleiche oder ungleiche linearen oder verzweigten, gesättigten oder ungesättigten Alkylrest mit 1 bis 23 C-Atomen. Besonders bevorzugt sind solche Diacylverbindungen der Formel (II) in denen die Reste R für linearen oder verzweigten, gesättigten oder ungesättigten Alkylrest mit 5 bis 17 und insbesondere für 9 bis 13 C-Atome steht.

Besonders bevorzugte Verbindungen der Formel (II) weisen als Reste R lineare gesättigte oder ungesättigte Alkylreste mit 1 bis 23 C-Atomen auf. Dabei sind solche Ester bevorzugt, die durch Umsetzung von Isosorbid mit kurzkettigen Fettsäuren mit 6 bis 12 C-Atomen hergestellt werden. Insbesondere bevorzugt sind solche Ester die auf Basis von C6, C8, C10 und/oder der C12-Fettsäuren mit Isosorbid erhalten werden. Weiterhin sind solche Derivate der Formel (II) bevorzugt, bei denen die Reste "R" für jeweils lineare und vorzugsweise gesättigte Alkylgruppen steht.

Diester von verzeigten Fettsäuren sind ebenfalls geeignet, wobei aber Diester des Isosorbids, die 2-Ethylhexyl-Reste weniger bevorzugt sind. Vorzugsweise können daher solche Mischungen, Zubereitungen oder Verwendungen vom Schutz ausgenommen sein, die einen Diester des Isosorbids mit 2-Ethylhexansäure enthalten.

Unabhängig davon sind solche Verbindungen der Formel (II) bevorzugt, bei denen die Reste R identisch sind, also ein symmetrischer Diester vorliegt.

Ein weiterer Gegenstand der vorliegenden Anmeldung betrifft die Verwendung von Ölkörpermischungen
(A) mindestens einen flüchtigen Kohlenwasserstoff und (B) mindestens einen Diacylester des Isosorbids der Formel (II) 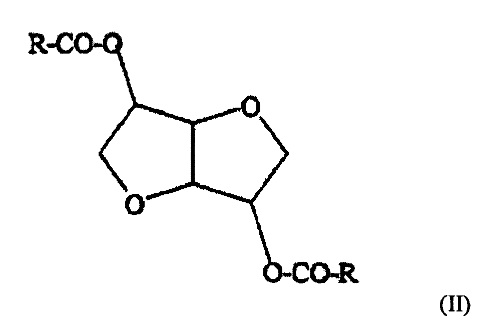
worin R unabhängig voneinander einen linearen oder verzweigten, gesättigten oder ungesättigten Alkylrest mit 1 bis 23 C Atomen darstellt in kosmetischen Zubereitungen. In einer bevorzugten Ausführungsform werden diese Mischungen als Silikonölersatz in kosmetischen Zubereitungen verwendet.

Bevorzugte Mischungen, Zubereitungen oder Verwendungen im Sinne der vorliegenden technischen Lehre benutzen als flüchtiger Kohlenwasserstoff (A) gesättigte und ungesättigte , lineare und verzweigte, cyclische Kohlenwasserstoffe mit einer C-Zahl von 6 bis 20. Vorzugsweise sind die Mischungen, Zubereitungen oder Verwendungen **dadurch gekennzeichnet, dass** in den Mittel bzw. Zubereitungen kein Mineralöl enthalten ist oder mit verwendet wird.

### Beispiele

Beispiel 1: Eine Ölkörpermischung bestehend aus 90 Gew.-% einer Mischung aus Undecan und Tridecan (70 : 30 w/w) und 10 Gew.-% eines Diacylesters (auf Basis von Octansäure) gemäß der allgemeinen Formel (I) durch Mischen der 2 Bestandteile hergestellt.

Der sensorische Vergleich dieser erfindungsgemäßen Mischung erfolgte gegen Cyclopentamethicone (DC 245, Fa. Dow Coming). Die erfindungsgemäße Ölkörpermischung zeigte eine mit Cyclopentamethicone vergleichbare Sensorik.

## Patentansprüche

1. Ölkörpermischung, enthaltend
(A) mindestens einen flüchtigen Kohlenwasserstoff und
(B) mindestens einen Diacylester des Isosorbids der folgenden Formel (I) 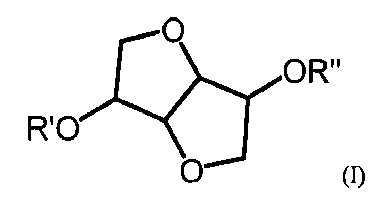
worin R' und R" unabhängig voneinander einen linearen oder verzweigten, gesättigten oder ungesättigten Acylrest mit 1 bis 24 C Atomen darstellt.

2. Mischung nach Anspruch 1, **dadurch gekennzeichnet, dass** sie 60 Gew.-% oder mehr, insbesondere 70 Gew.-% oder mehr, insbesondere 80 Gew.-% oder mehr, besonders bevorzugt 90 Gew.-% oder mehr, insbesondere 95 Gew.-% oder mehr der Komponenten (A) und (B) enthält.

3. Mischung nach einem der vorgenannte Ansprüche, **dadurch gekennzeichnet, dass** die Menge an (A) größer gleich 50 Gew.-% bezogen auf die Gesamtmenge von (A) und (B) beträgt, insbesondere größer gleich 60 Gew.-%, bevorzugt größer gleich 70 Gew.-%.

4. Mischung nach einem der vorgenannten Ansprüche, **dadurch gekennzeichnet, dass** sie weniger als 5 Gew.-%, insbesondere weniger als 3 Gew.-%, insbesondere weniger als 1 Gew.-% Silikonöle enthält.

5. Mischung nach einem der vorgenannten Ansprüche, **dadurch gekennzeichnet, dass** sie keine Silikonöle enthält.

6. Kosmetische Zubereitung, enthaltend
(A) mindestens einen flüchtigen Kohlenwasserstoff und
(B) mindestens einen Diacylester des Isosorbids

7. Kosmetische Zubereitungen nach Anspruch 6, enthaltend weniger als 5 Gew.-%, insbesondere weniger als 3 Gew.-%, insbesondere weniger als 1 Gew.-% Silikonöle.

8. Kosmetische Zubereitungen nach Anspruche 6 oder 7, **dadurch gekennzeichnet, dass** sie keine Silikonöle enthalten.

9. Kosmetische Zubereitung, nach mindestens einem der Ansprüche 6 bis 8, **dadurch gekennzeichnet, dass** die kosmetische Zubereitung neben (A) und (B) weniger als 40 Gew.-%, insbesondere weniger als 30 Gew.-%, insbesondere weniger als 20 Gew.-%, bevorzugt weniger als 10 Gew.-% weiterer lipophiler Komponenten enthält.

10. Verwendung von Ölkörpermischungen enthaltend
(A) mindestens einen flüchtigen Kohlenwasserstoff und
(B) mindestens einen Diacylester des Isosorbids der Formel (II) 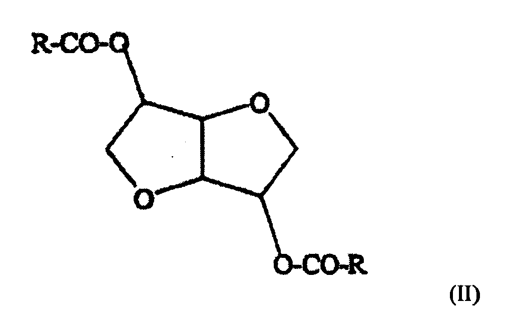
worin R unabhängig voneinander einen linearen oder verzweigten, gesättigten oder ungesättigten Alkylrest mit 1 bis 23 C Atomen darstellt in kosmetischen Zubereitungen.

11. Verwendung von Ölkörpermischungen nach mindestens einem der Ansprüche 1 bis 5 als Silikonersatz in kosmetischen Zubereitungen.

12. Mischung, Zubereitung oder Verwendung nach einem der vorgenannten Ansprüche, **dadurch gekennzeichnet, dass** als flüchtiger Kohlenwasserstoff (A) gesättigte und ungesättigte , lineare und verzweigte, cyclische Kohlenwasserstoffe mit einer C-Zahl von 6 bis 20 eingesetzt werden.

13. Mischung, Zubereitung oder Verwendung nach einem der vorgenannten Ansprüche, **dadurch gekennzeichnet, dass** kein Mineralöl enthalten ist oder mit verwendet wird.
